(19) Europäisches Patentamt
European Patent Office
Office européen des brevets

(11) Veröffentlichungsnummer: **0 036 389**
**A2**

(12) # EUROPÄISCHE PATENTANMELDUNG

(21) Anmeldenummer: 81810085.1

(22) Anmeldetag: 09.03.81

(51) Int. Cl.³: C 07 D 209/44
C 07 D 403/12, C 09 B 57/04
C 09 B 26/02

(30) Priorität: 13.03.80 CH 1979/80

(43) Veröffentlichungstag der Anmeldung: 23.09.81 Patentblatt 81/38

(84) Benannte Vertragsstaaten: CH DE FR GB IT LI

(71) Anmelder: CIBA-GEIGY AG
Patentabteilung Postfach
CH-4002 Basel(CH)

(72) Erfinder: Iqbal, Abul, Dr.
Im Schaiengarten 1
CH-4107 Ettingen(CH)

(54) **Metallkomplexe von Isoindolinazinen, Verfahren zu deren Herstellung und Verwendung.**

(57) 1:1-Metallkomplexe von Isoindolinazinen der Formel

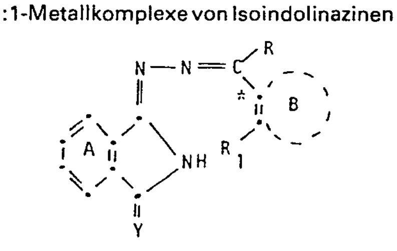

worin der Ring A noch weiter substituiert sein kann, R ein Wasserstoffatom, eine Alkyl- oder Arylgruppe, B einen isocyclischen oder heterocyclischen aromatischen Rest, $R_1$ die OH- oder SH-Gruppe und Y einen Rest der Formel

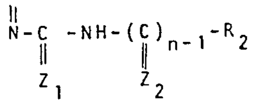 oder 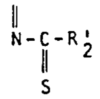

bedeutet, worin $Z_1$ und $Z_2$ für O- oder S-Atome, n für die Zahl 1 oder 2, $R'_2$ für einen Alkyl-, Aryl- oder Heteroarylrest steht, $R_2$ für einen Alkyl-, Cycloalkyl-, Aralkyl- oder Arylrest oder einen Rest der Formel

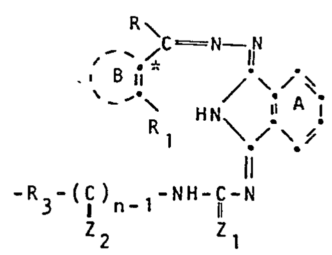

steht, worin $R_3$ eine Alkylen- oder Arylengruppe bedeutet.

Die neuen Pigmente färben Kunststoffe und Lacke in reinen farbstarken orangen bis roten Tönen von guten Echtheiten.

EP 0 036 389 A2

3-12759 +

Metallkomplexe von Isoindolinazinen, Verfahren zu deren Herstellung und Verwendung

Die Erfindung betrifft 1:1-Metallkomplexe von Isoindolinazinen der Formel

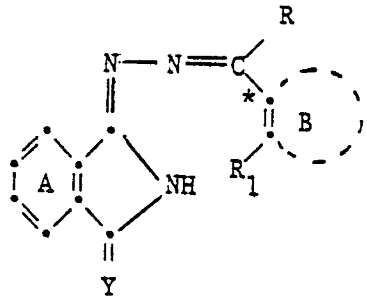

1)

worin der Ring A noch weiter substituiert sein kann, R ein Wasserstoffatom, eine Alkyl- oder Arylgruppe, B einen isocyclischen oder heterocyclischen aromatischen Rest, $R_1$ die OH- oder SH-Gruppe und Y einen Rest der Formel

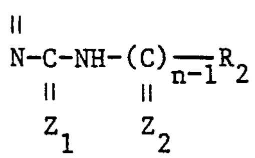

2) oder

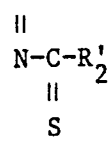

3)

bedeutet, worin $Z_1$ und $Z_2$ für O- oder S-Atome, n für die Zahl 1 oder 2, $R_2'$ für einen Alkyl-, Aryl- oder Heteroarylrest, $R_2$ für einen Alkyl-, Cycloalkyl-, Aralkyl- oder Arylrest oder einen Rest der Formel

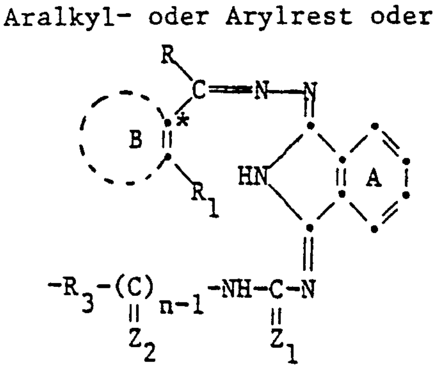

4)

steht, worin $R_3$ eine Arylengruppe bedeutet.

Die Isoindolinazine der Formel 1) können als Substituenten im Benzolrest A Halogenatome, beispielsweise 2-4 Chloratome, 1-2 Alkyl oder Alkoxygruppen mit 1-4 C, eine Phenyl-, Phenoxy-, Nitro- oder Benzoylaminogruppe oder eine Alkanoylaminogruppe mit 2-6 C enthalten, sind jedoch vorzugsweise unsubstituiert.

R steht beispielsweise für einen Phenyl- oder Naphthylrest, vorzugsweise für ein H-atom oder eine Alkylgruppe mit 1-4 C, insbesondere die Methylgruppe.

B bedeutet beispielsweise einen Phenylen- oder Naphthylenrest, insbesondere aber einen 5-6 gliedrigen Heteroring enthaltend ein zum C* Atom β-ständiges N- oder O-Atom und gegebenenfalls N, O oder S als weiteres Heteroatom und einen annelierten Benzol- oder Heteroring. B steht beispielsweise für einen Pyrazol-, Pyridin-, Pyrimidin-, Chinolin-oder Cumarinring. $R_1$ bedeutet vorzugsweise die Hydroxygruppe.

Steht $R_2$ oder $R_2'$ für einen Alkylrest, dann vorzugsweise für einen solchen mit 1-4 C. Bedeutet $R_2$ Cycloalkyl, dann insbesondere Cyclohexyl. Bedeutet $R_2$ oder $R_2'$ einen Arylrest, dann beispielsweise einen Naphthyl- und insbesondere Phenylrest. Bedeutet $R_2'$ einen Heteroarylrest, dann vorzugsweise einen Pyridin-, Pyridon-, Chinolin- oder Cumarinrest.

Bevorzugt sind Metallkomplexe der Formel

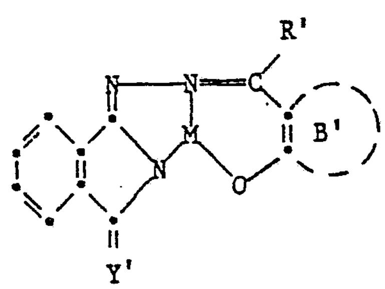

5)

worin M Nickel oder Kupfer, R' H oder Methyl, B' einen Pyrazol-, Pyrimidin-, Chinolin- oder Cumarinrest und Y' einen Rest der Formel

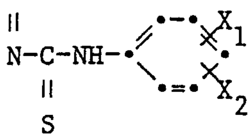 6),

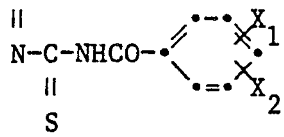 7) oder

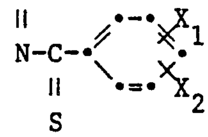 8)

bedeutet, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Phenylcarbamoyl- oder Phenylsulfamoylgruppe und $X_2$ ein H-, Chlor- oder Bromatom, eine Alkyl- oder Alkoxygruppe mit 1-4 C bedeuten.

Die Formeln 1) und 5) stellen eine der verschiedenen isomeren Formen dar.

Die Metallkomplexe der Formel 1) erhält man, wenn man
a) ein Azin der Formel 1) mit metallabgebenden Mitteln behandelt oder
b) ein Hydrazon der Formel

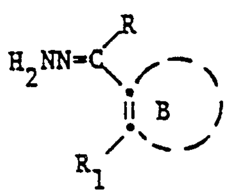 9)

worin R, $R_1$ und B die angegebene Bedeutung haben, mit einem Isoindolinon der Formel

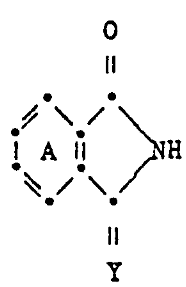 10)

oder einem Amino-isoindolamin der Formel

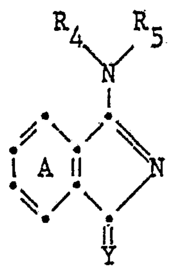

11)

worin $R_4$ und $R_5$ H-Atome, Alkyl-, Aryl- oder Heteroarylgruppen bedeuten oder $R_4$ und $R_5$ gemeinsam mit dem N-Atom einen heterocyclischen 5- oder 6-Ring bedeuten, A und Y die angegebene Bedeutung haben, in Gegenwart metallabgebender Mittel in einem polaren organischen Lösungsmittel erhitzt oder

c) ein Isoindolinazin der Formel

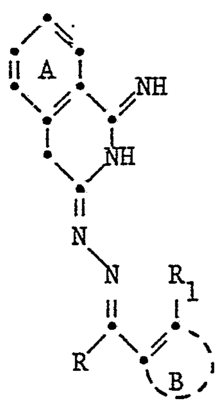

12)

worin A, R, $R_1$ und B die angegebene Bedeutung haben,
mit einem Thioamid der Formel

$$H_2N-\underset{\underset{S}{\parallel}}{C}-R'_2$$

worin $R'_2$ die angegebene Bedeutung hat, in Gegenwart von Metallsalzen in einem polaren organischen Lösungsmittel erhitzt.

Zu den Azinen der Formel 1) gelangt man beispielsweise nach dem in der GB-PS 1.467.595 beschriebenen Verfahren durch Kondensation eines Hydrazons der Formel 7) mit einem Amino-isoindolenin der Formel 9).

Die Verbindung der Formel 11) erhält man durch Umsetzung des Amino-imino-isoindolenins der Formel

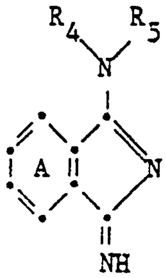

13)

worin A, $R_4$ und $R_5$ die angegebene Bedeutung haben, mit einer Verbindung der Formel

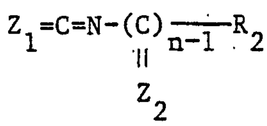

14)

worin $R_2$, $Z_1$, $Z_2$ und n die angegebene Bedeutung haben, insbesondere Isothiocyanaten der Formeln

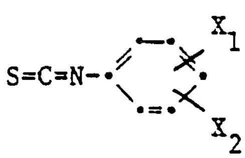

15) oder

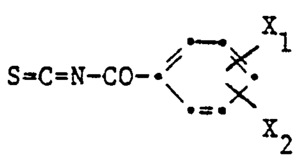

16)

worin $X_1$ und $X_2$ die angegebene Bedeutung haben.

Die Verbindung der Formel 13) ihrerseits erhält man durch Umsetzung des Amino-iminoisoindolenins der Formel

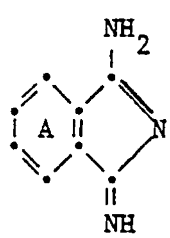

17)

mit einem Amin der Formel 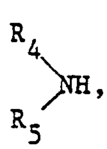, worin $R_4$ und $R_5$ die angegebene Bedeutung haben.

Die Verbindung der Formel 12) erhält man z.B. durch Umsetzung des Amino-iminoisoindolins der Formel 17) mit einem Hydrazon der Formel 9), worin A, B, R, $R_1$ die angegebene Bedeutung haben.

Sofern $R_4$ und $R_5$ Alkylreste bedeuten, dann vorzugsweise solche mit 1-6 C. Steht $R_6$ für einen Arylrest, dann vorzugsweise für einen gegebenenfalls durch Chloratome, Alkyl- oder Alkoxygruppen mit 1-4 C substituierten Phenylrest.

Als Beispiele von Amino-iminoisoindoleninen seien die auf S. 5 der GB-PS 1.465.595 aufgeführten erwähnt. Als Beispiele von Verbindungen der Formel 14) seien genannt:

Methyl-isocyanat
Aethyl- "
n-Propyl- "
Isopropyl- "
n-Butyl- "
Isobutyl- "
Cyclohexyl-"
Phenyl-isocyanat
N-o, m, oder p-Chlorphenyl-isocyanat
N-o, m- oder p-Methylphenyl- "
N-o, m- oder p-Methoxyphenyl- "
N-α-Naphthyl-isocyanat
Acetyl- "
Propionyl- "
Butyryl- "
Benzoyl- "
Methyl-isothiocyanat
Aethyl- "
n-Propyl- "
Isopropyl- "
n-Butyl- "

Tolyldiisothiocyanat
N-o-, m- oder p-Chlorbenzoyl-isothiocyanat
N-o-, m- oder p-Methylbenzoyl-isothiocyanat
N-α-Naphthoyl-isothiocyanat
N-o-, m- oder p-Chlorphenyl-isothiocyanat
N-o-, m- oder p-Methylphenyl-isothiocyanat
N-o-, m- oder p-methoxyphenyl-isothiocyanat
N-α-Naphthyl-isothiocyanat
1,4-Phenyl-diisocyanat
1,4-Phenyl-diisothiocyanat
Tolyldiisocyanat
Cyclohexyl-isothiocyanat
Phenyl-isothiocyanat

Die Hydrazone der Formel 9) erhält man nach bekanntem Verfahren durch Umsetzen einer Oxoverbindung der Formel

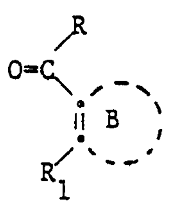

18)

worin R, $R_1$ und B die angegebene Bedeutung haben, oder deren Imin, insbesondere Anil, mit Hydrazinhydrat.

Als Beispiele von Oxoverbindungen der Formel 18) seien die auf S. 11 und 12 der GB-PS 1.467.595 aufgeführten Aldehyde und Ketone erwähnt, sowie 2-Formyl-5,5-dimethyl-cyclohexan-dion-1,3 oder 1-Phenyl-3-methyl-4-formyl-5-mercapto-pyrazol.

Die für den Weg b als Ausgangsstoffe dienenden Isoindolinone der Formel 10) erhält man durch Umsetzen der Imino-isoindolinone der Formel

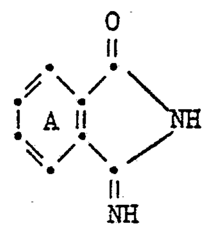

worin A die angegebene Bedeutung hat, mit Verbindungen der Formel 14).

Als metallabgebende Mittel verwendet man vorzugsweise die Salze des Zinks, Cadmiums, Mangans, Kobalts, Eisens, insbesondere aber Kupfers und des Nickels bzw. Mischungen solcher Metalle. Man verwendet zweckmässig die Formiate, Acetate oder Stearate dieser Metalle.

Die Umsetzungen erfolgen in einem polaren Lösungsmittel, insbesondere einem solchen hydrophiler Natur, beispielsweise einem Amid wie Dimethylformamid, Formamid, Dimethylacetamid oder N-Methylpyrrolidon, ferner Dimethylsulfoxid, Acetonitril oder einem Alkohol wie beispielsweise Aethylcellosolve. Es kann auch ein Gemisch polarer Lösungsmittel verwendet werden.

Die Umsetzungstemperatur liegt zweckmässig zwischen 100-200°C.

Die Isolierung des erhaltenen Metall-Komplexes erfolgt wie üblich durch Filtration. Das Nutschgut wird mit Lösungsmittel gut gewaschen. Es wird in ausgezeichneter Ausbeute und Reinheit erhalten und kann ohne weitere Reinigung in feinverteilter Form zum Färben von hochmolekularem organischem Material verwendet werden, z.B. Celluloseäthern und -estern, wie Aethylcellulose, Acetylcellulose, Nitrocellulose, Polyamiden bzw. Polyurethanen oder Polyestern, natürlichen Harzen oder Kunstharzen, z.B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyäthylen, Polypropylen, Polyacrylnitril, Polyacrylsäureester, thermoplastische und härtbare Acrylharze, Gummi, Casein, Silikon und Silikonharzen, einzeln oder in Mischungen. Die erwähnten hochmolekularen Verbindungen können als plastische Massen, Schmelzen oder in Form von Spinnlösungen, Lacken oder Druckfarben vorliegen. Je nach Verwendungszweck erweist es sich als vorteilhaft, die neuen Pigmente als Toner oder in Form von Präparaten zu verwenden.

Die Pigmente können in der Form, wie sie in der Synthese anfallen, oder in leicht gemahlener Form eingesetzt werden und liefern dann opake Ausfärbungen. Man kann sie aber auch einer intensiveren Mahlung unterziehen und erhält dann transparente Ausfärbungen, wie beispielsweise farbstarke Metalleffektlackierungen.

Anreibungen der Pigmente in Lacken zeichnen sich durch ein günstiges Fliessverhalten aus.

Die erhaltenen Färbungen beispielsweise in Kunststoffen, Fasern und Lacken zeichnen sich durch grosse Farbstärke, hohe Farbtonreinheit, gute Verteilung der Pigmente, gute Ueberlackier-, Migrations-, Hitze-, Licht- und Wetterechtheit sowie durch einen guten Glanz aus.

In den nachfolgenden Beispielen bedeuten die Prozente Gewichtsprozente und die Grade Celsiusgrade.

Beispiel 1: 1,09 g (0,005 Mol) 3-Acetyl-4-hydroxycumarinhydrazon und 1,31 g (0,00525 Mol) Nickelacetat·$4H_2O$ werden in 40 ml Aethylcellosolve angeschlämmt. Man erwärmt die Suspension auf 60°C und gibt anschliessend 1,41 g (0,005 Mol) 1-Phenylthiocarbamoyliminoisoindolinon-3, hergestellt aus Phenylisothiocyanat und 1-Iminoisoindolinon-3, hinzu. Das Reaktionsgemisch wird nun auf 120°C erhitzt und bei derselben Temperatur 2 Stunden gerührt. Der Metallkomplex fällt so dick aus, dass zwischendurch mit 40 ml Aethylcellosolve verdünnt werden muss. Nach Ablauf der Reaktionszeit von 2 Stunden kühlt man auf 100°C ab und filtriert heiss. Das Nutschgut wird mit Aethylcellosolve und Sprit nachgewaschen und über Nacht unter Vakuum bei 80°C getrocknet. Man erhält 2,5 g (92,9% der Theorie) eines 1:1-Nickelkomplexes der Formel

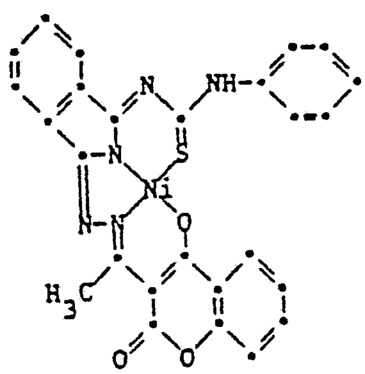

Elementaranalyse: $C_{26}H_{17}N_5O_3SNi$ MG 538.23

| | | | | | |
|---|---|---|---|---|---|
| ber. | C 58,02% | H 3,18% | N 13,01% | S 5,96% | Ni 10,91% |
| gef. | C 58,00% | H 3,1 % | N 13,1 % | S 6,2 % | Ni 10,9% |

Das obige Pigment färbt Kunststoffe und Lacke in roten Tönen von ausgezeichneten Echtheiten.

Beispiel 2: Man schlämmt 1,09 g (0,005 Mol) 3-Acetyl-2,4-dihydroxychinolinhydrazon und 1,31 g (0,00525 Mol) Nickelacetat·$4H_2O$ in 40 ml Aethylcellosolve an, erwärmt die resultierende Suspension auf 60°C und gibt anschliessend 1,55 g (0,005 Mol) 1-Benzoylthiocarbamoylimino-3-oxo-isoindolin, hergestellt aus Benzoylisothiocyanat und 1-Imino-3-oxo-isoindolin, hinzu. Das Reaktionsgemisch wird unter Rühren auf 120°C erhitzt und bei derselben Temperatur 2 h gerührt. Nach Ablauf der Reaktionszeit wird auf 100°C abgekühlt und heiss filtriert. Man wäscht das Nutschgut mit Aethylcellosolve und Aethanol nach und trocknet über Nacht unter Vakuum bei 80°C. Auf diese Weise erhält man 2,3 g (81% der Theorie) eines roten Metallkomplexes der Formel

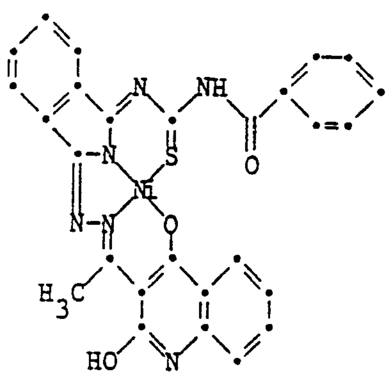

Mikroanalyse: $C_{27}H_{18}N_6O_3SNi$ MG 565

| | | | | | |
|---|---|---|---|---|---|
| ber. | 57,34% C | 3,18% H | 14,86% N | 5,66% S | 10,39% Ni |
| gef. | 57,7% C | 3,1% H | 14,9% N | 5,7% S | 10,6% Ni |

Das obige Pigment färbt Kunststoffe und Lacke in roten Tönen von ausgezeichneten Echtheiten.

Beispiele 3-15 Analog den Beispielen 1 und 2 erhält man weitere 1:1-Nickelkomplexe, wenn man das Hydrazon der in Kolonne 2 der Tabelle 1 angegebenen Oxoverbindungen mit dem Isoindolinon der Formel

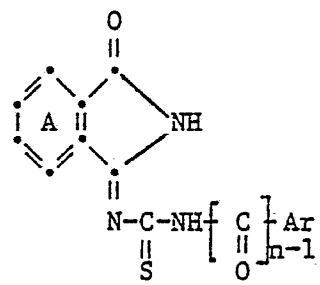 (n = 2 oder 1)

kondensiert, welch letzteres erhalten wurde durch Kondensation des in Kolonne 3 erwähnten 3-Imino-isoindolinon mit der in Kolonne 4 aufgeführten Isothiocyanat-Verbindung. Kolonne 5 gibt die Nuance in PVC.

Tabelle 1

| Beispiel Nr. | Oxoverbindung | Isoindolinon | $S=C=N-\left[\underset{\underset{O}{\|}}{C}\right]_{n-1}-Ar$ (n =2 od. 1) | Nuance in PVC |
|---|---|---|---|---|
| 3 | 2-Phenyl-5-acetyl-4,6-dihydroxy-pyrimidin | 1-Imino-isoindolinon-3 | Phenylisothiocyanat | orange |
| 4 | 3-Acetyl-2,4-dihydroxy-chinolin | do. | do. | rot |
| 5 | 5-Acetyl-2,4,6-trihydroxy-pyrimidin | do. | do. | orange |
| 6 | 1-p-Chlorphenyl-3-methyl-4-acetyl-pyrazolon-5 | do. | do. | rot |
| 7 | do. | do. | do. | rot |
| 8 | 3-Acetyl-2,4-dihydroxy-chinolin | do. | p-Chlor.benzoyl-isothiocyanat | rot |
| 9 | do. | do. | p-Tolylisothio-cyanat | rot |
| 10 | do. | do. | p-Chlorphenyl-isothiocyanat | rot |
| 11 | 3-Acetyl-4-hydroxy-cumarin | do. | 3,4-Dichlor-phenyl-isothiocyanat | orange |
| 12 | do. | do. | p-Methoxyphenyl-isothiocyanat | scharlach |
| 13 | do. | do. | p-Methoxybenzoyl-isothiocyanat | orange |
| 14 | 1-Phenyl-3-methyl-4-formyl-5-mercapto-pyrazol | do. | 3-Chlor-4-methyl-benzoylisothiocyanat | rot |
| 15 | 3-Acetyl-4-hydroxy-cumarin | 1-Imino-5,6-dichloro-isoindolinon -3 | 3-Trifluormethyl-phenylisothiocyanat | rot |

Beispiel 16:. 2,07 g (0,006 Mol) der Verbindung der Formel

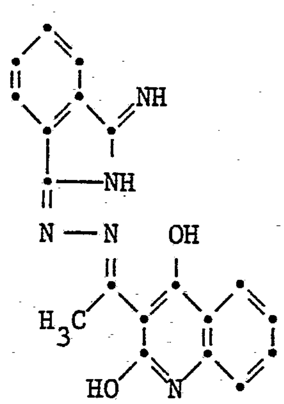

hergestellt aus 1,3-Diiminoisoindolin und 3-Acetyl-2,4-dihydroxy-chinolinhydrazon, 1,5 g (0,006 Mol) Nickelacetat. $4H_2O$ und 0,85 g (0,006 Mol) Thiobenzamid,werden in 60 ml Dimethylformamid angeschlämmt und anschliessend auf 110°C erhitzt. Man lässt das Gemisch während 1 h bei derselben Temperatur ausreagieren. Hierauf wird auf 80°C abgekühlt und filtriert. Das Nutschgut wird mit Dimethylformamid und Aethanol gewaschen und über Nacht bei 80°C unter Vakuum getrocknet.

Man erhält 2,82 g (90% der Theorie) des 1:1-Nickelkomplexes der Formel (nur eine der möglichen isomeren bzw. tautomeren Formen wurde berücksichtigt)

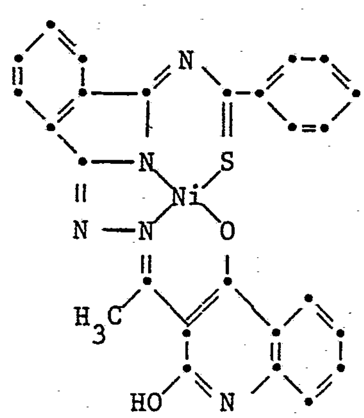

als braunes Pulver.

Mikroanalyse: $C_{26}H_{17}N_5O_2SNi$ Mol.-Gew. 522,2

| | | | | | |
|---|---|---|---|---|---|
| ber. | 59.80% C | 3,28% H | 13,41% N | 6,14% S | 11,24% Ni |
| gef. | 59,46% C | 3,5% H | 13,82% N | 5,7% S | 11,3% Ni |

Der obige Komplex färbt Kunststoffe und Lacke in braunen Tönen von ausgezeichneten Echtheiten.

Beispiel 17: Verwendet man in Beispiel 16 anstelle des Thiobenzamids Thioacetamid, so erhält bei analoger Arbeitsweise den 1:1-Nickelkomplex der folgenden Zusammensetzung (nur eine der möglichen isomeren bzw. tautomeren Formen wurde berücksichtigt)

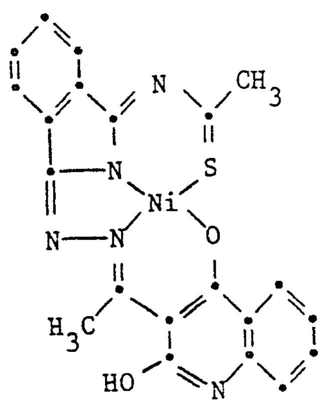

als rotbraunes Pulver.

Mikroanalyse: $C_{21}H_{15}N_5O_2SNi$ Mol.-Gew. 460,2

| | | | | | |
|---|---|---|---|---|---|
| ber. | 54.81% C | 3,29% H | 15,22% N | 6,97% S | 12,76% Ni |
| gef. | 54,6% C | 3,5% H | 15,5% N | 6,7% S | 12,6% Ni |

Das obige Metallkomplex-Pigment färbt Kunststoffe und Lacke in braunen Tönen von hohem Echtheitsniveau.

Beispiel 18: Man schlämmt 1,09 g (0,005 Mol) 3-Acetyl-2,5-dihydroxychinolinhydrazon und 1,31 g (0,00525 Mol) Nickelacetat. $4H_2O$ in 100 ml Dimethylformamid an, erwärmt die resultierende Suspension auf 60°C und gibt anschliessend 1,66 g 1-α-Naphthylthiocarbamoylimino-3-oxo-isoindolin, hergestellt aus 2-Naphthylisothiocyanat und 1-Imino-3-oxoisoindolin, hinzu. Das Reaktionsgemisch wird unter rühren auf 120°C erhitzt und bei derselben Temperatur 2 Stunden gerührt. Nach Ablauf der Reaktionszeit wird auf 100°C abgekühlt und heiss filtriert. Man wäscht das Nutschgut mit Dimethylformamid und Aethanol nach und trocknet über Nacht unter Vakuum bei 80°C. Auf diese Weise erhält man 2,8 g (96% der Theorie) eines roten Metallkomplexes der Formel

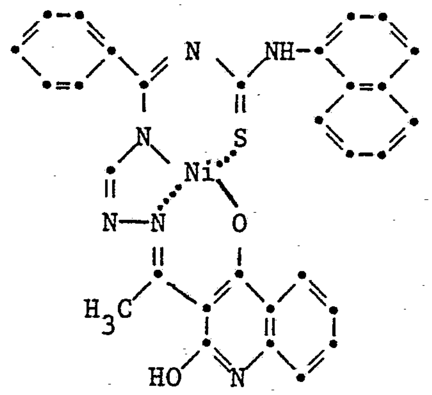

Mikroanalyse: $C_{30}H_{20}N_6O_2SNi$ MG 587

| | | | | | |
|---|---|---|---|---|---|
| ber.: | 61,4% C | 3,4% H | 14,3% N | 5,5% S | 9,7% Ni |
| gef.: | 61,3% C | 3,1% H | 14,5% N | 6,1% S | 10,0% Ni . |

Beispiele 19-24: Analog dem Beispiel 18 erhält man weitere 1:1 Nickelkomplexe der Formel

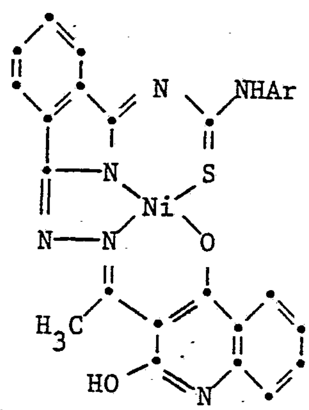

wenn man 3-Acetyl-2,4-dihydroxy-chinolin-hydrazon in Gegenwart von Nickelacetat mit dem Isoindolinon der Formel

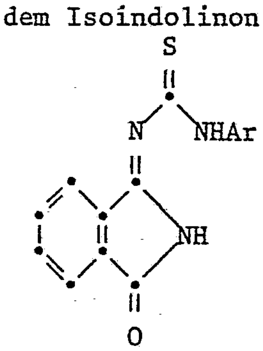

kondensiert, welch letzteres erhalten wurde durch Kondensation von 1-Imino-3-oxo-isoindolin mit der in Kolonne 2 aufgeführten Isothiocyanat-Verbindung. Kolonne 3 gibt die Nuance der Metallkomplexe in PVC wieder.

| Beispiel Nr. | SCN-Ar | Nuance in PVC |
|---|---|---|
| 19 | m-Trifluormethyl-phenylisothiocyanat | blaurot |
| 20 | p-Nitrophenyl-isothiocyanat | blaurot |
| 21 | p-Phenoxy-phenyl-isothiocyanat | blaurot |
| 22 | p-Acetylamino-phenylisothiocyanat | scharlach |
| 23 | 4'-Chlorbenzoyl-amino-phenylisothio-cyanat | scharlach |
| 24 | Benzoylisothiocyanat | rot |

Beispiel 25: 1,58 g (0,005 Mol) der Verbindung der Formel

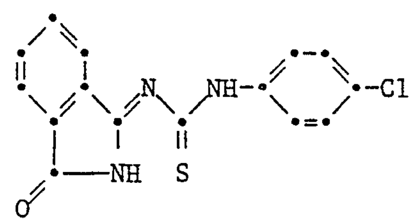

hergestellt aus 1-Imino-3-oxo-isoindolin und p-Chlorphenylisocyanat, 1,31 g (0,00525 Mol) Nickelacetat.$4H_2O$, sowie 0,92 g (0,005 Mol) 5-Acetyl-2,4,6-trihydroxypyrimidin-hydrazon werden in 70 ml Aethylcellosolve gut angerührt und auf 110°C erhitzt. Man rührt bei derselben Temperatur $2\frac{1}{2}$ Stunden lang, kühlt das Gemisch anschliessend auf 80°C ab und filtriert. Das Nutschgut wird mit Aethylcellosolve und Aethanol gewaschen und über Nacht bei 80°C unter Vakuum getrocknet. Man erhält 2,6 g (96,5%) des 1:1 Metallkomplexes der folgenden Zusammensetzung (nur eine der möglichen tautomeren bzw. isomeren

Formen wird berücksichtigt) als rötlich oranges Pulver:

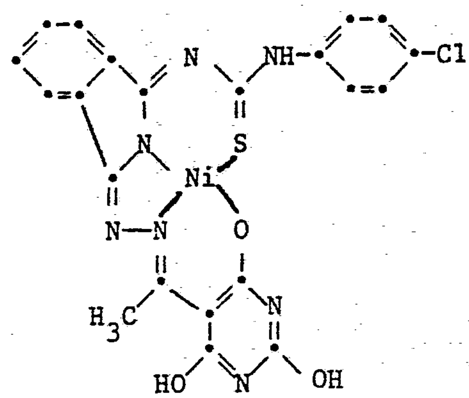

Mikroanalyse: $C_{21}H_{14}ClN_7O_3SNi$ Mol.-Gew. 538,6

| | | | | | | |
|---|---|---|---|---|---|---|
| ber.: | 46,83% C | 2,62% H | 18,20% N | 5,95% S | 6,58% Cl | 10,90 % Ni |
| gef.: | 46,5% C | 2,6% H | 18,3% N | 5,9% S | 6,4% Cl | 11,0% Ni |

Der obige Metallkomplex färbt Kunststoffe und Lacke in reinen orangen Tönen von ausgezeichneten Echtheiten.

Beispiel 26: In einem Laboratoriums-Kneter von 250 Vol.-Teilen Inhalt legt man 25 Teile des gemäss Beispiel 2 hergestellten Pigmentes, 100 Teile feingemahlenes Natriumchlorid und 30 Teile Diacetonalkohol vor. Man knetet die Mischung während 5 Stunden unter Kühlung und trägt sie dann in 4000 Vol-Teile Wasser ein. Natriumchlorid sowie Diacetonalkohol gehen in Lösung, das Pigment fällt aus. Man filtriert die Suspension, wäscht das Nutschgut gründlich mit Wasser und trocknet es im Vakuumtrockenschrank bei 80°.

Beispiel 27: 65 Teile stabilisiertes Polyvinylchlorid, 35 Teile Dioctylphthalat und 0,2 Teil des gemäss Beispiel 16 erhaltenen Pigmentes werden miteinander verrührt und dann auf einem Zweiwalzenkalander während 7 Minuten bei 160° hin- und hergewalzt. Man erhält eine rot gefärbte Folie von sehr guter Licht- und Migrationsechtheit.

Beispiel 28: 10 g Titandioxyd und 2 g des nach Beispiel 2 hergestellten Pigments werden mit 88 g einer Mischung von 26,4 g Kokosalkydharz, 24,0 g Melamin-Formaldehydharz (50% Festkörpergehalt), 8,8 g Aethylenglykolmonomethyläther und 28,8 g Xylol während 48 Stunden in einer Kugelmühle vermahlen.

Wird dieser Lack auf eine Aluminiumfolie gespritzt, 30 Minuten bei Raumtemperatur vorgetrocknet und dann während 30 Minuten bei 120°C eingebrannt, dann erhält man eine Rotlackierung, die sich bei guter Farbstärke durch eine sehr gute Ueberlackier-, Licht- und Wetterechtheit auszeichnet.

Beispiel 29: 4 Teile des fein verteilten Pigments gemäss Beispiel 16 werden in 20 Teilen Lösungsmittel der folgenden Zusammensetzung eingerührt: 50 Teile Solvesso 150 (Gemisch aromatischer Kohlenstoffe), 15 Teile Butylacetat, 5 Teile Exkin II (Verlaufmittel auf Ketoximbasis), 25 Teile Methyl-Isobutylketon, 5 Teile Silikonöl (1% in Solvesso 150).

Nachdem die vollständige Feinverteilung erreicht ist (je nach Art des Rührens in ca. 15-60 Minuten), werden die Bindemittel zugesetzt, nämlich 48,3 Teile Baycryl L 530 (Acrylharz) (51% in Xylol/Butanol 3:1) und 23,7 Teile Maprenal TTX (Melaminharz) (55% in Butanol).

Nach kurzem Homogenisieren wird der Lack nach üblichen Methoden wie Spritzen und Tauchen oder speziell zur kontinuierlichen Beschichtung von Metallblechen im "Coil-Coating"-Verfahren appliziert und eingebrannt (Einbrennen 30 Minuten, 130°). Die erhaltenen roten Lackierungen zeichnen sich aus durch sehr guten Verlauf, hohen Glanz und ausgezeichnete Feinverteilung des Pigmentes, sowie durch ausgezeichnete Wetterechtheiten.

Beispiel 30: Verfährt man wie in Beispiel 16 beschrieben, fügt jedoch der Knetmasse 2,78 Teile Staybelite Resin (HERCULES) zu, so erhält man ein Pigment, enthaltend 10% Harz, das sich auszeichnet durch leichtere Einarbeitbarkeit und bessere Verteilbarkeit.

Patentansprüche

1. 1:1-Metallkomplexe von Isoindolinazinen der Formel

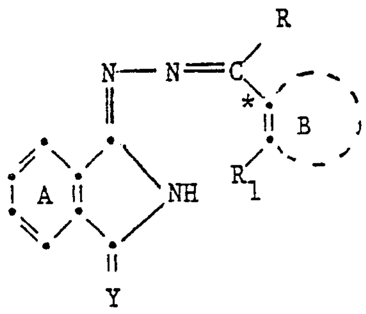

1)

worin der Ring A noch weiter substituiert sein kann, R ein Wasserstoffatom, eine Alkyl- oder Arylgruppe, B einen isocyclischen oder heterocyclischen aromatischen Rest, $R_1$ die OH- oder SH-Gruppe und Y einen Rest der Formel

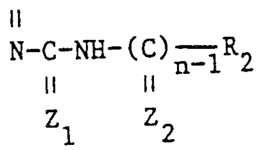

2) oder

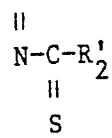

3)

bedeutet, worin $Z_1$ und $Z_2$ für O- oder S-Atome, n für die Zahl 1 oder 2, $R_2'$ für einen Alkyl-, Aryl- oder Heteroarylrest, $R_2$ für einen Alkyl-, Cycloalkyl-, Aralkyl- oder Arylrest oder einen Rest der Formel

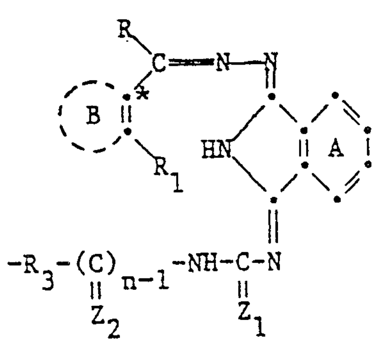

4)

steht, worin $R_3$ eine Alkylen- oder Arylengruppe bedeutet.

2. Metallkomplexe der Formel

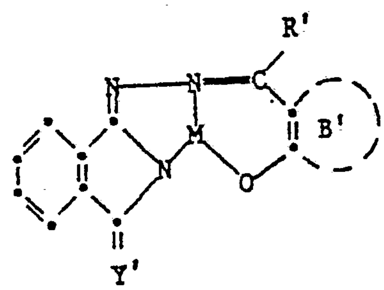

5)

worin M Nickel oder Kupfer, R' H oder Methyl, B' einen Pyrazol-, Pyridin-, Pyrimidin-, Chinolin- oder Cumarinrest und Y' einen Rest der Formel

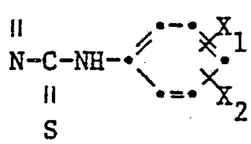

6),

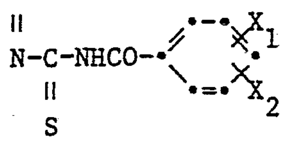

7) oder

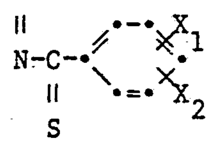

8)

bedeutet, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C-Atomen, eine Alkanoylamino-, Alkylcarbamoyl- oder Alkoxycarbonylgruppe mit 2-6 C, eine gegebenenfalls durch Chlor- oder Bromatome oder Methylgruppen substituierte Phenoxy-, Phenylcarbamoyl- oder Phenylsulfamoylgruppe und $X_2$ ein H-, Chlor- oder Bromatom, eine Alkyl- oder Alkoxygruppe mit 1-4 C bedeuten.

3. Verfahren zur Herstellung von 1:1-Metallkomplexen gemäss Anspruch 1, dadurch gekennzeichnet, dass man

a) ein Azin der Formel 1) mit metallabgebenden Mitteln behandelt oder

b) ein Hydrazon der Formel

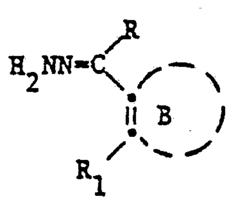

9)

worin R, $R_1$ und B die in Anspruch 1 angegebene Bedeutung haben, mit einem Isoindolinon der Formel

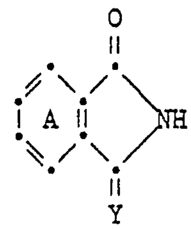

10)

oder einem Amino-isoindolenin der Formel

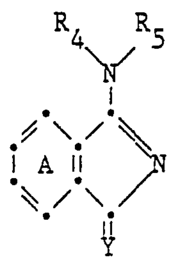

11)

worin A und Y die in Anspruch 1 angegebene Bedeutung haben, $R_4$ und $R_5$ H-Atome, Alkyl-, Aryl- oder Heteroarylgruppen bedeuten oder $R_4$ und $R_5$ gemeinsam mit dem N-Atom einen heterocyclischen 5- oder 6-Ring bedeuten, in Gegenwart metallabgebender Mittel in einem polaren organischen Lösungsmittel erhitzt oder c) ein Isoindolinazin der Formel

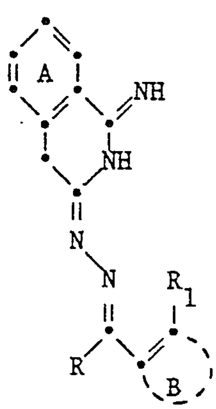

12)

worin A, R, $R_1$ und B die angegebene Bedeutung haben, mit einem Thioamid der Formel

$$H_2N-\underset{\underset{S}{\parallel}}{C}-R'_2$$

worin $R'_2$ die angegebene Bedeutung hat, in Gegenwart von Metallsalzen in einem polaren organischen Lösungsmittel erhitzt.

4. Verfahren gemäss Anspruch 3, dadurch gekennzeichnet, dass man von einem Isoindolinon der Formel 10) oder einem Aminoisoindolenin der Formel 11) ausgeht, worin Y einen Rest der Formel

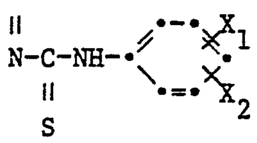 6), 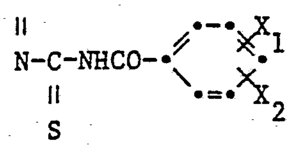 7) oder 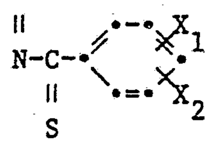 8)

bedeutet, worin $X_1$ und $X_2$ die in Anspruch 2 angegebene Bedeutung haben.

5. Verfahren gemäss Anspruch 3, dadurch gekennzeichnet, dass man von einem Hydrazon der Formel 12) ausgeht, worin B einen Pyrazol-, Pyridin-, Pyrimidin-, Chinolin- oder Cumarinrest bedeutet.

6. Verfahren gemäss Anspruch 3, dadurch gekennzeichnet, dass man als metallabgebende Mittel Nickelsalze verwendet.

7. Verfahren zum Färben von hochmolekularem organischem Material, gekennzeichnet durch die Verwendung der Metallkomplexe gemäss Anspruch 1.

8. Hochmolekulares organisches Material enthaltend einen Metallkomplex gemäss Anspruch 1.